(19) Europäisches Patentamt / European Patent Office / Office européen des brevets

(11) **EP 4 479 558 B1**

(12) **EUROPEAN PATENT SPECIFICATION**

(45) Date of publication and mention of the grant of the patent:
**04.02.2026 Bulletin 2026/06**

(21) Application number: **23708000.7**

(22) Date of filing: **17.02.2023**

(51) International Patent Classification (IPC):
***C12Q 1/686*** (2018.01)

(52) Cooperative Patent Classification (CPC):
(C-Sets available)
**C12Q 1/686** (Cont.)

(86) International application number:
**PCT/GB2023/050361**

(87) International publication number:
**WO 2023/156784 (24.08.2023 Gazette 2023/34)**

(54) **METHODS, SYSTEMS AND DEVICES FOR QPCR AND ARRAY-BASED ANALYSIS OF TARGETS IN A SAMPLE VOLUME**

VERFAHREN, SYSTEME UND VORRICHTUNGEN FÜR QPCR UND ARRAYBASIERTE ANALYSE VON ZIELMOLEKÜLEN IN EINEM PROBENVOLUMEN

PROCÉDÉS, SYSTÈMES ET DISPOSITIFS POUR LA QPCR ET L'ANALYSE BASÉE SUR UN RÉSEAU DE CIBLES DANS UN VOLUME D'ÉCHANTILLON

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.02.2022 GB 202202224**

(43) Date of publication of application:
**25.12.2024 Bulletin 2024/52**

(73) Proprietor: **QuantuMDx Group Limited**
**Newcastle upon Tyne, Tyne and Wear NE1 2JQ (GB)**

(72) Inventors:
• **GOWDY, James Alexander**
**Newcastle upon Tyne, Tyne and Wear NE1 2JQ (GB)**
• **PLAZA GARAY, Nerea**
**Newcastle upon Tyne, Tyne and Wear NE1 2JQ (GB)**

(74) Representative: **Definition IP Limited**
**The Core**
**Newcastle Helix**
**Bath Lane**
**Newcastle Upon Tyne NE4 5TF (GB)**

(56) References cited:
**WO-A1-2009/156895      WO-A1-2010/119396**
**WO-A2-2006/135437      US-A1- 2019 262 832**

EP 4 479 558 B1

(52) Cooperative Patent Classification (CPC): (Cont.)

C-Sets
**C12Q 1/686, C12Q 2537/101, C12Q 2537/143,
C12Q 2561/113, C12Q 2565/519, C12Q 2565/629**

**Description**

**Technical Field**

The present invention relates to methods, systems and devices which allow the rapid analysis of multiple nucleic acid targets within a single sample volume using both real-time and array-based detection.

**Background**

In recent years there have been ongoing efforts to develop and manufacture microfluidic devices, such as cassettes, that are able to perform various chemical and biochemical analyses and syntheses 'on device'. The goal is to provide devices that allow previously laboratory-based activities to be performed at or near the point of care (POC) in a timely and efficient manner. Such microfluidic devices can be adapted for use with automated systems, thereby providing the additional benefits such as cost reductions and decreased operator errors.

In many cases it is desirable that such microfluidic devices are capable of carrying out molecular techniques including the amplification of nucleic acids.

Polymerase chain reaction (PCR) amplification techniques are an important cornerstone of molecular biology. Since it's conception, PCR amplification has been used to analyse and research nucleic acid sequences. Conventional polymerase chain reaction (PCR) methods amplify a single target sequence in a sample. As is now well known, a pair of primers specific to the target sequence, along with a number of specific reactants including a polymerase, dNTPs and cations such as magnesium, are exposed to thermal cycling to produce large numbers of copies (amplicons) of the target sequence.

Real time PCR (also known as quantitative PCR or qPCR) allows the amplification of target sequences to be monitored in real time as it incorporates fluorescent dyes into the amplified targets (amplicons) and a fluorometer is used to detect the resulting fluorescence. Fluorescence can be measured after every cycle and the intensity of the fluorescent signal reflects the amount of amplicon present at that point. This can be useful as it can allow a very quick identification of the presence of a target sequence in a sample as the presence can be identified at the point at which the fluorescence intensity increases above background fluorescence. This can also provide a level of quantification as the point at which the fluorescence intensity increases above the detectable level corresponds proportionally to the initial number of template DNA molecules in the sample.

Multiplex PCR reactions are now being used to allow the simultaneous amplification of multiple target sequences. Multiplex qPCR reactions are also possible. Multiple primer sets, each set specific to a different target sequence, are used in combination with probes which are labelled with spectrally distinct fluorophores. However the limited number of fluorophore labels available, and the overlap in their emission spectra, does mean that multiplex PCR can be challenging to set up and the number of targets that can be analysed is limited.

Microarrays (often referred to as gene arrays, DNA chips or biochips) can be used to screen a lot of gene targets simultaneously. A large number of different nucleic acid or oligonucleotide probes (oligos) are attached to a solid surface. The probes will hybridise under stringent conditions to cDNA or cRNA targets. Probe/target hybridisation is typically detected as the targets are labelled with a fluorescent dye incorporated during target amplification. After the microarray is washed only bound targets will be detectable and the location of signal allows the bound target to be identified.

Whilst the above techniques have been used for many years in laboratories, more recently point of care (POC) devices have been developed which have allowed molecular techniques to be carried out in systems with microfluidic devices such as chips, cartridges or cassettes in near to patient settings or similar. Such systems allow a range of molecular techniques, including PCR amplification and analysis, to be carried out 'on-cassette' with minimal or no user interaction. For example, US2019/0262832 describes a microfluidic device such as a 'lab on a chip' device which can be configured to allow a plurality of different analysis or detection methods to be carried out. Whilst powerful, there are challenges in the design of POC systems and microfluidic devices with space and cost being important factors to allow these systems to be usable on a larger scale.

**Summary of the Invention**

According to a first aspect of the present invention there is provided a method for analysing a plurality of nucleic acid targets in a single sample volume by:

amplifying at least one primary nucleic acid target in a reaction volume using real-time polymerase chain reaction (qPCR) and monitoring the resulting primary amplicon generation in real-time;
and
amplifying a plurality of different secondary nucleic acid targets in the same reaction volume using polymerase chain reaction and analysing the resulting secondary amplicons on a microarray,
wherein the amplification of the at least one primary nucleic acid target and the amplification of the plurality of secondary nucleic acid targets occurs substantially simultaneously in the same reaction volume.

The inventors identified that it would be useful to use both qPCR and array-based analysis on samples to provide both rapid, quantifiable and more detailed results. However, this to date has required separate reactions to be carried out. When looking at point of care diagnostics in particular this results in challenges regarding space on the microfluidic devices that are used and cost implications.

The terms 'primary' nucleic acid target or amplicon and 'secondary' nucleic acid targets or amplicons do not necessarily designate any relative importance (e.g. diagnostic importance) of the targets. However, in some cases the primary target(s) may be selected to allow the presence or absence of a particular pathogen to be identified and/or quantified, with the secondary target(s) providing further particularisation of the pathogen.

Advantageously, this allows the amplification of the primary and secondary nucleic acid targets to be carried out in a single reaction volume, which requires less volumetric space than if the qPCR and array PCR methods were done separately as would usually be the case. This can be particularly useful if running the method on a microfluidic device such as a cassette or cartridge. It also requires less reagents such as buffers and/or dNTPs which has cost benefits.

Optionally, where the target is an ribonucleic acid (RNA) target, the method can include the step of reverse transcription of RNA to DNA prior to amplification.

Preferably, a plurality of different primary nucleic acid targets are amplified in a reaction volume to give a plurality of groups of different primary amplicons. In a preferred embodiment, up to five primary nucleic acid targets in a reaction volume are amplified in this step to give up to five primary amplicon groups.

A 'group of amplicons' or 'amplicon group' being a set of amplicons with the same sequence/ which have been expanded from the same target sequence. Effectively the 'group of amplicons' or 'amplicon group' are product(s) of a PCR reaction(s) being multiple copies of the same amplicon sequence.

Preferably, in the step of amplifying a plurality of primary nucleic acid targets each resulting group of primary amplicons is labelled with a different fluorophore and the method includes the step of detecting fluorescence emission of each of the fluorophores upon excitation.

Preferably, in the step of amplifying a plurality of primary nucleic acid targets each resulting group of primary amplicons is detected through the use of 'probes', each probe having a different set of fluorophore and quencher attached, with the method including a final step of detecting fluorescence emission of each of the fluorophores upon excitation.

In the case of qPCR (with fluorometer, also referred to as in-line reader, based detection) the primary amplicon is not labelled directly with the fluorophore, it's 'labelled' indirectly with its presence being detected by the binding of a probe, said probe having a sequence complimentary to a part of the amplicon sequence and which has both fluorophore and quencher attached. Binding of the probe to the amplicon sequence then allows hydrolysis of the probe by a DNAse (part of the polymerase) which separates/releases fluorophore and quencher - leading to fluorescence.

Each primary amplicon is detectable using fluorescently labelled oligonucleotide probes complementary to said amplicons.

Preferably, the fluorescently labelled oligonucleotide probes complementary to said amplicons are labelled with a quencher.

When the probe is intact, the fluorescence of the fluorescent label is quenched due the proximity of the quencher to the fluorescent label.

Preferably each primary amplicon group is labelled with a different fluorophore. The labelling in this case in indirect as the fluorophores are covalently attached to primary target probes (as are quenchers), said probes being cleaved (and de-quenched) during the amplification process.

Preferably when monitoring the resulting primary amplicon generation in real-time, the detection of primary amplicons is carried out at least once every thermocycle. Alternatively detection could occur after a predetermined number of thermocycles.

The plurality of secondary nucleic acid targets are amplified by an asymmetric PCR.

Pairs of secondary primers are used for the amplification of each of the secondary nucleic acid targets. Either the forward or reverse primer in each pair is provided in excess.

Preferably the amplicon groups expanded from the plurality of secondary nucleic acid targets are all labelled with the same fluorophore. The fluorophore used to label the plurality of secondary nucleic acid target amplicons is different to the one or more fluorophores used to label the one or more primary nucleic acid targets.

Preferably, at least one of each primer pair used to amplify the plurality of secondary nucleic acid targets are labelled with the same fluorophore. This results in labelled secondary amplicons. Preferably the primer that is in excess is the labelled primer.

Preferably the fluorophore used to label the plurality of secondary nucleic acid targets is Cy5 or UV fluorophore AF350.

All currently known fluorophores have shorter emission wavelengths than AF350 and its emission spectra is far removed from FAM/HEX/ROX/Cy5/AF750. For Cy5, only AF750 has a longer emission wavelength, so being less 'central' makes it a good choice for labelling the plurality of secondary nucleic acid targets. Cy5 is also a good choice as the associated excitation wavelengths for this fluorophore are known to have minimal risk of causing plastics used in the microarray insert to auto-fluoresce in the UV region

Optionally, when the fluorophore used to label the plurality of secondary nucleic acid targets is Cy5, fluorophores used to label primary nucleic acid targets may be one or more selected from AF350, FAM and HEX. ROX can also be used.

Optionally the fluorophores used to label primary nucleic acid targets may be one or more selected from AF350, FAM, HEX or ROX.

Preferably the fluorophore used to label the plurality of secondary nucleic acid targets is selected to have an excitation spectrum that has limited or no overlap with the excitation spectra of the fluorophores used to label primary nucleic acid targets.

More particularly, the fluorophore used to label the plurality of secondary nucleic acid targets is selected to have an excitation spectrum that has limited or no overlap with the excitation spectra of the fluorophores used to label primary nucleic acid targets within the region of the excitation filter bandwidth specific to the primary nucleic acid target fluorophores.

Preferably, the fluorophore used to label the plurality of secondary nucleic acid targets is selected to have an emission spectrum that has limited or no overlap with the emission spectrums of the fluorophores used to label primary nucleic acid targets.

More particularly, the fluorophore used to label the plurality of secondary nucleic acid targets is selected to have an emission spectrum that has limited or no overlap with the emission spectra of the fluorophores used to label primary nucleic acid targets within the region of the emission filter bandwidth specific to the primary nucleic acid target fluorophores.

Advantageously, filter sets comprising emission filters and excitation filters can be used to ensure the emission crosstalk is less than 10% of the total signal, preferably less than 5% of the total signal, preferably less than 1% of the total signal, preferably less than 0.01% of the total signal.

Effectively the maximum wavelength of absorption and maximum wavelength of emission of the fluorophore used to label the plurality of secondary nucleic acid targets is spectrally separated (in terms of difference in wavelength) from the other wavelengths of the fluorophores used to label primary nucleic acid targets. As such, two or less, preferably one or less, most preferably none, have bleed-through characteristics that make them unusable.

As the fluorophore used to label the plurality of secondary nucleic acid targets will often be in excess compared to any of the fluorophores used to label primary nucleic acid targets, and will be unquenched (unlike the fluorophore used to label the primary target probe which will, at least at the start of the reaction be quenched), the fluorescence attributable to the fluorophore used to label the plurality of secondary nucleic acid targets will be high compared to any of the fluorophores used to label primary nucleic acid targets. The fluorophore used to label the plurality of secondary nucleic acid targets is also often selected to have a high extinction coefficient and fluorescent quantum yield to allow for ease of imaging.

Optionally, the fluorophore used to label the plurality of secondary nucleic acid targets is selected to have an emission spectrum lower or higher than all of the emission spectrums of the fluorophores used to label primary nucleic acid targets.

Optionally, the fluorophore used to label the plurality of secondary nucleic acid targets is selected to have an emission spectrum bandwidth that is spectrally located at a lower or higher wavelength distinct from all others that are being used.

Optionally, the fluorophore used to label the plurality of secondary nucleic acid targets is selected to be excitable at a wavelength that has limited or no overlap with the excitation wavelength spectrum of the fluorophores used to label primary nucleic acid targets.

Preferably the fluorophore used to label the plurality of secondary nucleic acid targets is selected to have an excitation spectrum that has limited or no overlap with the excitation spectrums of the fluorophores used to label primary nucleic acid targets.

Preferably, the fluorophores used to label primary nucleic acid targets are selected to be excitable at wavelengths that have limited or no overlap with the excitation wavelength spectrum of the fluorophore used to label the plurality of secondary nucleic acid targets.

Preferably, the fluorophore used to label the plurality of secondary nucleic acid targets is selected to either have: an emission spectrum peak and associated bandwidth at a longer wavelength than all, or most, of fluorophores used to probe the primary nucleic acid target sequences, or else at a bandwidth short enough and sufficiently spectrally separated, so that the long-wavelength emission spectrum tail of the secondary target fluorophore overlaps with the minimum number of emission spectra.

The above approach minimizes crosstalk from the fluorophores used to label the plurality of secondary nucleic acid targets in the detection channels of the fluorophores used to probe the primary nucleic acid targets. As such two or less, preferably one or less, most preferably none, of the primary target fluorophore channels will experience crosstalk from the secondary target fluorophore making them either unusable due to saturation, or greatly reducing their detection channels dynamic range.

Crosstalk refers to light emission from one fluorophore being detected in the detection channel of another fluorophore.

As dynamic range is not excessively reduced or lost on the qPCR channels specific to the primary nucleic acid target probes, with a constant background due to the secondary nucleic acid target primer-probe, a combined multiplex qPCR - microarray test is possible in the same reaction volume. Furthermore as the amplification of the secondary amplicon does not result in an increasing signal despite crosstalk characteristics the combination of assay technologies will not increase the risk of false positive results, for the primary nucleic acid targets.

According to another aspect of the present invention there is provided a microfluidic device, and qPCR and asymmetric PCR reagents, for carrying out the method of the first aspect comprising;

a body section with at least one microchannel;
means for moving a liquid sample through the microchannel;
the microchannel having an amplification area in which PCR amplification is performed, said area comprising heating portion and a cooling portion through which a sample can cycle, and a first detection window through which fluorescence emission from the sample can be detected during every amplification cycle;
the microchannel having an array area, downstream of the amplification area, said array area comprising a second detection window through which fluorescence emission can be detected.

The amplification area is a portion of the channel that has been configured to allow thermocycling of a sample passing therethrough to occur. The PCR section of the channel is specifically adapted to allow a liquid sample travelling therethrough to be heated and cooled in a cyclical manner to temperatures that allow for denaturing of DNA, annealing of DNA and amplification of DNA. The PCR section of the channel is heatable either by an integral heat source within the channel or the wall of the device, or more typically by being brought into the proximity of an external heat source which applies heat to microfluidic device (such as a microfluidic cassette) in a manner that allows heat to transfer into the PCR section of the channel.

Optionally, at least part of the microchannel in the amplification area has a serpentine configuration.

Advantageously serpentine configurations can allow long fluid flow in a relatively small footprint, which means an efficient heat transfer by increasing surface to volume ratio.

In some embodiments the serpentine configuration can be useful in allowing fluid to travel over multiple different temperature zones, for example different heat plates, before curving back to repeat the process. In other embodiments the serpentine configuration can be utilised along with methods of shuttling fluid back and forth between temperature zones (i.e. shuttle flow (shuttle PCR). It is preferred that device is configured for shuttle flow PCR.

Optionally the device may include onboard reagents.

Optionally onboard reagents may be one or more of:

- A polymerase, preferably a thermostable polymerase e.g., *Taq* polymerase;
- A mixture of dNTPs;
- A buffer;
- At least one pair of primary amplification primers;
- At least a first hybridisation probe labelled with a first fluorophore;
- A plurality of pairs of secondary amplification primers at least one of each pair being labelled with a second fluorophore;
- A source of Magnesium ions e.g., Magnesium Chloride;
- A reverse transcriptase (where the target is RNA).

According to another unclaimed aspect of the present invention there is provided a system for carrying out the method of the first aspect comprising;

the microfluidic device of the second aspect; and
a host instrument able to receive the microfluidic device.

Preferably the host instrument comprises a microprocessor and software which controls the interactions between the host instrument and the microfluidic device.

Preferably the host instrument comprises a heater which heats the heating portion of the microfluidic device when the microfluidic device is in the host device.

Preferably the host instrument comprises a fluorometer and/or an imager.

Preferably the host instrument comprises a excitation source. The excitation source being a fluorescence excitation source or an excitation light source.

The fluorometer and/or imager can be arranged, as is known in the art, to detect and analyse particular emission wavelengths. Associated software can be used to analyse the detected fluorescence data.

Various further features and aspects of the invention are defined in the claims.

Unless defined otherwise, all technical and scientific terms used herein have the meaning commonly understood by a person skilled in the art to which this invention belongs.

RT-PCR (reverse-transcription PCR) is a variation of PCR, or polymerase chain reaction which allows amplification and analysis of RNA targets. The techniques are generally the same except that RT-PCR has an added step of reverse transcription of RNA to DNA, to allow for amplification. Throughout this document the term PCR can be assumed to encompass RT-PCR unless explicitly indicated to the contrary or it would be clear that the steps required for RT-PCR are not being included.

The term 'Shuttle flow' or 'Shuttle PCR' refers to techniques in which thermocycling is performed by shuttling small plugs of PCR mixture back and forth between temperature zones. In this way temperature variations occur spatially.

Throughout this document reference to "microfluidic" means with at least one dimension less than 1 millimetre and/or able to deal with microlitre or less portions of fluid.

"Oligonucleotide" means a molecule which comprises a chain of nucleotides. Oligonucleotides are commonly in the form of DNA or ribonucleic acid (RNA). The term "oligonucleotide" includes polynucleotides of genomic DNA or RNA, complementary DNA (cDNA), and polynucleotides of semisynthetic, or synthetic origin. Standard nucleotide bases may also be substituted with nucleotide isoform analogs. In this context the term "oligonucleotide" is somewhat interchangeable "polynucleotide" although generally refers to nucleotide chains of shorter length.

Throughout this document reference to "cassette" or "chip" means an assembled unit comprising one or more substrates with channels or chambers therein through which fluid can flow. Such cassettes may include different regions or zones in which activities such as sample mixing, filtering, PCR amplification, reverse-transcription, identification and/or visualisation can occur and may include on-board reagents. The cassettes are typically designed to be received by a diagnostic instrument such as a point-of-care (POC) instrument which incorporates additional functionality to allow a diagnostic test, or part of such a test, to be automated.

The term "fluorophore" used herein means a functional group and/or a molecule containing said functional group which will absorb energy (light energy) of a specific wavelength and re-emit energy (light energy) at a different (longer) wavelength.

The term "fluorometer" refers to a device, which measures the intensity of light that is generated by a sample as it fluoresces that has a different wavelength than the light projected into the sample. The fluorescence light can be measured at an angle with respect to the light projected into the sample. The device may be referred to as an in-line reader or a fluorescence reader in this document.

As used herein in relation to PCR, the expression "real-time" means that the polymerase chain reaction can be monitored as it progresses and without halting or opening the reaction vessel. This can be by continuous detection or be regular or semi-regular detection. By monitoring how the amplification occurs and in particular at which cycles exponential increase in amplicon becomes significant allows the amount of target nucleic acid present in the sample being subject to the PCR to be quantitated as is well known and understood in the art.

Reference to "reaction volume" is to the volume of fluid sample, preferably liquid sample, in which biochemical reactions such as PCR are occurring.

## Brief Description of the Drawings

Embodiments of the present invention will now be described by way of example only with reference to the accompanying drawings, where like parts are provided with corresponding reference numerals and in which:

Figure 1 shows a sectional plan view of a microfluidic cassette, showing select microfluidic features, according to an aspect of the present invention;

Figure 2 shows an indicative spectra of a fluorophore multiplex system illustrating spectral overlaps, from the fluorometer of a 6-channel multiplex system. Emission spectra are filled, excitation spectra are not;

Figure 3 is a graph showing the PCR trace of the FAM channel showing commencement of PCR curve during the running of an HPV assay in accordance with an aspect of the present invention;

Figure 4 is an image of the microarray post hybridisation showing alignment spots (faint) and target spots HPV 16 and HPV 18 (brighter);

Figure 5a is a schematic representation of an in-line reader (ILR) (also referred to as a fluorometer) showing the different optical components and their spectral response at each step of the optical pathway.

Figure 5b is a cross- section through a model of an in-line reader (ILR) (also referred to as a fluorometer) showing the physical set-up of the one light path for the qPCR.

Figure 6a is a schematic representation of an in-line reader (ILR) (also referred to as a fluorometer) showing the different optical components and their spectral responses for two fluorophores at each step of the optical pathway.

Figure 6b shows normalized spectra of all the optical components in two channels (Cy5 and AF750) for a possible combination of components. Alignment of excitation filters and emission filters with their respective fluorophore's excitation and emission spectra, while also misaligning to some extent with the opposing fluorophores excitation and emission spectra is shown.

Figure 6c Shows the resulting emission spectra scaled according to the total radiant power absorbed when exited by the channel LED, with the emission intensity within the emission bandpass that will contribute to the total signal indicated by block colour.

Figure 7 shows qPCR curves with crosstalk from off channel fluorophores used to probe primary target sequences (7a and 7b) and qPCR curves with crosstalk from off channel fluorophores used to label the secondary target sequences (7c and 7d)

## Detailed Description

The invention will now be described with reference to some specific examples.

*STI testing method*

An exemplary sexually transmitted infection (STI) testing method will now be described for identifying the presence of gonorrhoea and/or Chlamydia trachomatis (CT) pathogens in a sample, and further particularising those pathogens if they present to provide information about the strain present and potential antimicrobial resistance (AMR). Antimicrobial resistance in Neisseria gonorrhoeae and Chlamydia trachomatis is a major public health concern so rapid information about the appropriate treatment at the point of care is of great importance.

A PCR reaction is set up in a single reaction volume. The following reagents are included in the sample volume:

a cDNA or DNA target or template (10ng -100ng gDNA which may be diluted);
a first set of primary primers which amplify a part of *Neisseria gonorrhoeae* genome to give *N. gonorrhoeae* primary amplicons;
a second set of primary primers which amplify a part of *Chlamydia trachomatis* genome to give *C. trachomatis* primary amplicons;
a first set of primary target probes to *N. gonorrhoeae* primary amplicons, said probes labelled with AF350 (Alexa Fluor 350) fluorophore;
a second set of primary target probes to *C. trachomatis* primary amplicons, said probes labelled with FAM fluorophore;
multiple pairs of secondary forward and reverse primers which amplify multiple parts of *Neisseria gonorrhoeae* genome to give *N. gonorrhoeae* secondary amplicons, where at least one of the secondary primers in each pair is labelled with Cy5 fluorophore;
multiple pairs of secondary forward and reverse primers which amplify multiple parts of *Chlamydia trachomatis* genome to give *C. trachomatis* secondary amplicons, where at least one of the secondary primers in each pair is labelled with Cy5 fluorophore;
a DNA polymerase such as Taq DNA polymerase;
dNTPs;
$MgCl_2$;
buffers such as salt buffers.

In this embodiment, the primary target probes are also labelled with a quencher. Each probe is labelled with a FAM or AF350 reporter at the 5' end and a 'Black Hole Quencher 1' dye quencher at the 3' end. When the probe is intact, the fluorescence of the FAM or AF350 is quenched due to its proximity to the quencher. However, during a PCR amplification reaction, which has a combined annealing/extension step, the probe hybridizes to the target, and the dsDNA-specific 5' → 3' exonuclease activity of the polymerase cleaves off the reporter. As a result, the reporter is separated from the quencher, resulting in a fluorescence signal that is proportional to the amount of amplified product in the sample.

The pairs of secondary forward and reverse primers are provided in relative amounts appropriate for asymmetric PCR. One primer in each secondary primer pair is provided in excess and this is used to preferentially amplify one strand of the target DNA more than the other. The primer in excess is labelled with Cy5 fluorophore.

Sets of primary target probes and primary primers are provided in a 1:1 - 1:2 ratio.

In this embodiment the primary primers amplify sections of the target pathogens that are well conserved, to allow identification of the general pathogen.

Each set of secondary primers is provided at a similar concentration to each set of primary primers. However, as there are multiple sets of secondary primers and each one is labelled with Cy5 (c.f. primary probes where each set is labelled with a different fluorophore) the fluorophores involved in labelling the secondary primers, and ultimately the secondary amplicons, will be significantly in excess of other fluorophores.

Even if the concentration of primary probes were increased, there can still be potential knock out issues with the secondary primer signal. The secondary primer (e.g. Cy5) signal is amplicon concentration *independent,* the other signals are (indirectly) amplicon concentration *dependent.* Even if PCR is 100% efficient the concentration of the cleaved and unquenched fluorophore and hence associated signal will not reach that of the unquenched secondary primer (in this case Cy5) signal.

In this embodiment, the secondary primers amplify sections of the target pathogens that are indicative of particular strains or which have sequence variations which been associated with antibiotic resistance.

Whilst the above reaction mix is appropriate for a DNA target it would be understood that a RNA target could also be used by setting up a reverse transcription reaction. This could be carried out prior to the above or concurrently in the same reaction mix as would be well understood by those skilled in the art.

Master mixes specifically formulated for multiplexing are available commercially and typically incorporate appropriate amounts of a DNA polymerase such as Taq DNA polymerase, dNTPs, $MgCl_2$ and buffers. It is generally understood that a multiplex reaction will require at least twice as much polymerase as the minimum amount previously titrated for a singleplex qPCR. Appropriate levels of dNTPs are required to ensure it does not limit the reaction so would generally be added in excess. The increased total amount of nucleic acid in the reaction (e.g., from adding additional dNTPs as well as primers/probe, or by generating more template) also requires the $Mg^{2+}$ concentration to be added in an amount the will not limit the reaction - this is well understood by those in the art when optimising qPCR reactions.

Once the PCR reaction is set up, the well understood thermal cycling reaction is carried out.

An example of a two-step reaction using a FAST polymerase would be e.g. 40 cycles of:

| Conditions | Temperature (°C) | Time (sec) |
|---|---|---|
| Step 1 Denaturing | 95 | 5 (first cycle 30) |
| Step 2 Annealing and Extending | 60 | 30 |

and/or a 3 step reaction could be used e.g.:

| Conditions | Temperature (°C) | Time (sec) |
|---|---|---|
| Step 1 Denaturing | 95 | 5 (first cycle 30) |
| Step 2 Annealing | 58 | 15 |
| Step 3 Extending | 72 | 10 |

but it is well understood that the steps can be optimised as required.

A fluorometer detects fluorescence from the primary probes in real-time, which in this embodiment using a two-step PCR is at a regular interval every cycle after step 2. If either *Neisseria gonorrhoeae* or *Chlamydia trachomatis* are present the fluorescence from AF350 or FAM will increase with every cycle. As each probe fluorophore was selected to fluoresce at a different frequency from others that may be present in the reaction it is possible to identify which primary targets are present and gain some understanding or relative and absolute amounts of target present in the sample.

Although the Cy5 will be present in significantly higher amounts than either of AF350 or FAM, as the excitation and emission wavelengths do not overlap it is still possible to identify the AF350 or FAM. Similarly, further primary probes could be labelled with HEX or ROX and still be read in real time against the background of Cy5.

The amplified mix is also applied to a spotted microarray which has a plurality of secondary probes attached to a solid support in a manner well known in the art. The secondary probes are designed to hybridise under stringent conditions any *N. gonorrhoeae* secondary amplicons and C. *trachomatis* secondary amplicons present in the amplified sample. These secondary amplicons having been labelled with Cy5. After washing any unbound material from the support, any Cy5 fluorescence detected on the array can be identified.

*STI testing panel*

There are now a variety of tests available for use at or near the point of patient care (POC) for STIs allowing for the provision of cost effective and rapid tests that would allow the rapid indication of the presence of a pathogen along with further particularising of that pathogen, if present, to assist with treatment choices has real benefits.

In an exemplary embodiment of the present invention, a point-of-care version of the above described STI test is provided.

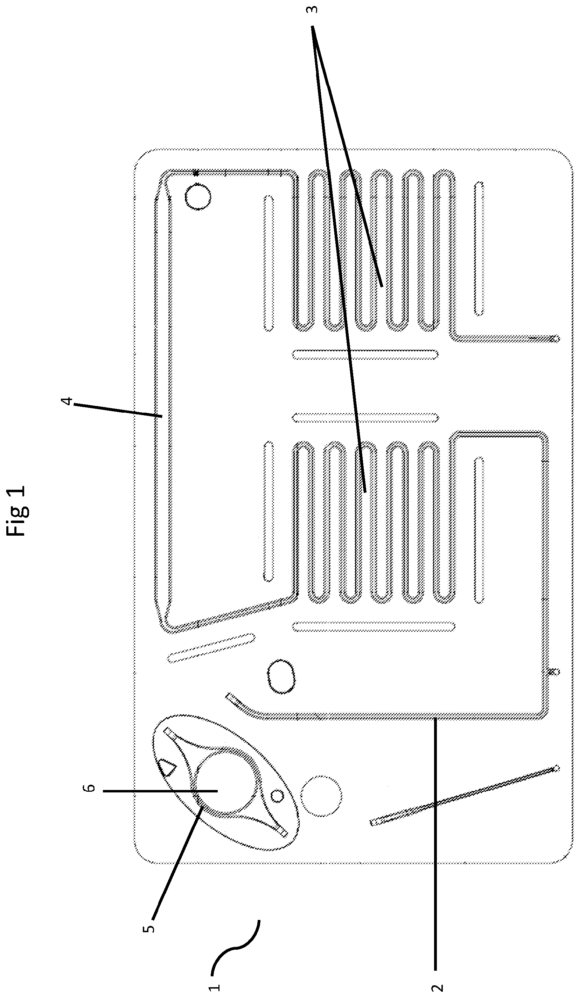

As generally depicted in figure 1, there is provided a microfluidic cassette 1, more generally termed a 'device', with a microchannel 2, where the microchannel 2 allows for continuous flow-through of fluid as required.

The microchannel 2 is formed in a first surface of a first substrate, typically a substantially planar, substantially rigid substrate which in this embodiment is polypropylene. The first substrate is overlaid with a second substrate, which in this embodiment is a polypropylene film. By bonding the first substrate material to the film, for example using laser welding, a substantially closed channel 2 is provided (inlets and outlets can be included as required). It will be understood that if the first substrate is a planar element with an upper and lower surface, the majority of the microchannel 2 can formed in the upper surface or the lower surface. It is however often desirable that the second substrate, i.e. the film, forms the upper wall of the microchannel 2 in use. The use of laser welding ensured that a fluid-tight, and more specifically an air-tight seal is created around the microchannels, such that no air can escape or leak out between the first and second substrates. It will be understood that methods other than laser welding can be used and will typically be selected based on the substrate materials themselves. The seal or weld is sufficiently strong to withstand increased pressure levels within the cassette compare to outside of the cassette. Typically, it is strong enough to withstand internal gauge pressures of at least 2bar.

It would be understood that although this embodiment has the second substrate as a film, the second substrate can be another material and may itself have grooves or channel formed on its surface that can be aligned with the channels of the first substrate. By bonding the substrates together, a closed channel is provided (again inlets and outlets can be included as required). Where necessary, the first and second substrates can be aligned prior to bonding. The length and cross-sectional shape of the channel can be any appropriate shape to allow for the desired transport and processing of a sample and or reagents.

Various valves and fluidically intercommunicating offshoots such as additional channels, reservoirs or chambers can be used to allow mixing, washing, removal and other actions to occur according to the needs of a diagnostic or biochemical assay to be performed therein in an automated or semi-automated manner. The method is generally carried out by allowing a sample to interact and/or react with one or more reagents in one or more steps; typically in one or more channels or chambers of the device, for times and at temperatures effective in forming a detectable product that indicates the presence or absence of an analyte in the sample.

The micro-channel 2 is formed inside the microfluidic cassette 1, in the desired length and shape to allow the passage of a sample, typically a biological sample in liquid format, and/or reagents, some of which may be incorporated on-cassette during the flow-through, along a fluid flow path and through various zones or areas which allow different activities to occur including amplification of DNA from the sample by polymerase chain reaction (PCR). A PCR or amplification section of the channel 3 is a portion of the channel that has been configured to allow thermocycling of a sample passing therethrough to occur. The PCR section 3 of the channel (and cassette) is specifically adapted to allow a liquid sample travelling therethrough to be heated and cooled in a cyclical manner to temperatures that allow for denaturing of DNA, annealing of DNA and simplification of DNA. The PCR section 3 of the channel is heatable either by an integral heat source within the channel or the wall of the device, or more typically by being brought into the proximity of an external heat source which applies heat to microfluidic device (such as a microfluidic cassette) in a manner that allows heat to transfer into the PCR section 3 of the channel. In this embodiment, the PCR section 3 is configured to shuttle sample to and fro (e.g. repeatedly oscillatory, or in a downstream direction followed by an upstream direction) between heating and cooling zones. A first detection window **4** is provided in the PCR section at a point on the microchannel which allows the cycling sample to be exposed to one or more excitation wavelengths and emission from the sample detected. Detection is typically by an appropriate imager and/or fluorometer.

The external heat source, excitation source and imager and/or fluorometer are typically provided as part of the host analyser instrument which receives and interacts with the cassette to perform an assay or test. The host instrument can also carry many of the actuators, imagers, fluorometers as well as software and microprocessors required to automate testing methods. Such microfluidic systems 'lab-on-a-chip' type systems are well known in the art.

Downstream of, and in fluid communication with, the PCR section 3 of the microchannel 2 is a spotted array 5. The array 5 has spotted oligonucleotides on a solid support. The array 5 can be provided as a separate plug that can be incorporated into the cassette during or after its manufacture. The array is associated with a second detection window 6 that allows any fluorescence from the array to be detected and/or imaged. Detection may be by the same imager and/or fluorometer as for the first detection window, or by a second imager and/or fluorometer.

The cartridges are typically consumables; i.e., they are used once and then discarded; and contain all or many of the reagents needed for one or more assays to be performed. Such reagents can be held on the cartridge in reservoirs or similar. In this embodiment the cartridge has both wet and dry onboard reagents that are required for PCR reactions including a thermostable DNA polymerase; a mixture of dNTPs; buffer; primers and probes as described in the above method. Where the cassette is to be used for an RNA target it can also have reverse transcriptase held onboard. There are various mechanisms known in the art for holding wet or dry reagents on a cassette and releasing them into the relevant potion of the microfluidic channel as needed.

### HPV testing

An exemplary at or near point-of-care (POC) human papillomavirus (HPV) test has been developed. The test allows for both real time and array-based detection of multiple nucleic acid targets within a single sample volume, in this case to assist with the detection of HPV and the identification of HPV subtypes.

A microfluidic cassette of the type described in the above POC STI panel was provided. Again there is a microchannel with a PCR section; the PCR section having a detection window at a point on the microchannel which allows the cycling sample to be exposed to one or more excitation wavelengths, and emission from the sample detected in real time by a spectrofluorometer or reader. The spectrofluorometer or reader can detect fluorescence and/or fluorescence intensity and has multiple channels such that multiple different emissions across different emission bands can be detected. See 'Additional Comments' below for more details.

In this specific example the cassette was a Q-POC™ cassette developed by QuantumDx™ for use with their Q-POC™ rapid multiplex PCR platform and device.

The cassettes used RIPA buffer spiked with a sample containing:

| - Human Genomic DNA (female) | 40,000 copies/ml | = for detection by ILR (qPCR) |
| - HPV 16 Plasmid (end-point PCR) | 100,000 copies/ml | = for detection by microarray |
| - HPV 18 Plasmid (end-point PCR). | 100,000 copies/ml | = for detection by microarray |

(these are only 'exemplars' and the system would also be applicable to clinical samples having target concentrations both greater and less than those described)

**Primer/Probe Details**

| Target | Sequence | Conc. ($\mu$M) |
|---|---|---|
| Human Beta Globin (HBB) HBB01F | (21mer) | 0.4 |
| Human Beta Globin (HBB)HBB01R | (23mer) | 0.4 |
| Human Beta Globin (HBB)HBB_P36_FAM_ZEN | (FAM-9mer-ZEN-14mer-3IABkFQ) | 0.4 |
| HPV 16127F_HPV16F | (20mer) | 0.2 |
| HPV 16121R_HPV16R_Cy5 | (Cy5-21mer) | 1.0 |
| HPV 1805F4_18_HPV18F | (21mer) | 0.2 |
| HPV 185R_HPV18/45R_Cy5 | (Cy5-21mer) | 1.0 |

Microarray Probe Details

| Target | Name | Sequence |
|---|---|---|
| Human Beta Globin (HBB) | GX088 Control | (5AmMC12//iSp18//iSp18-19mer) |

| | | |
|---|---|---|
| HPV 16 | GX075 | (5AmMC12//iSp18//iSp18-20mer) |
| HPV 18 | GX001 | (5AmMC12//iSp18//iSp18-22mer) |
| Alignment Probe | GG110 | (5AmMC12//iSp18//iSp18-Cy5-30mer) |

On cassette the sample was eluted in 375mM Trehalose + 0.1% Pluronic. 50uL was used to rehydrate the PCR/microarray reagents:

| Reagent | Final Concentration in 50μl | |
|---|---|---|
| Lyo-Ready 1-Step-qPCR Reaction Mix, | 0.9 | x |
| Glycerol-Free Taq HS, | 0.45 | U/μl |
| Taq Antibody, | 0.045 | mg/mL |
| Enzyme Dilution Buffer (10x) | 0.09 | x |
| Magnesium Chloride | 1.0 | mM |
| Trehalose Solution (50%) | 1.17 | % |
| Molecular Grade Water | - | - |

400ul of the sample described above was pipetted into a cassette and the cassette placed in the Q-POC™ instrument.

The sample was processed, all on cassette.

DNA was purified from the sample by capture on to a silica membrane filter.

Wash steps (70% ethanol) were carried out to remove contaminants then eluted in 100ul of 375mM Trehalose + 0.1% Pluronic Elution Buffer of which 50ul was then used to resuspend the PCR reagents (see above) contained within the cassette.

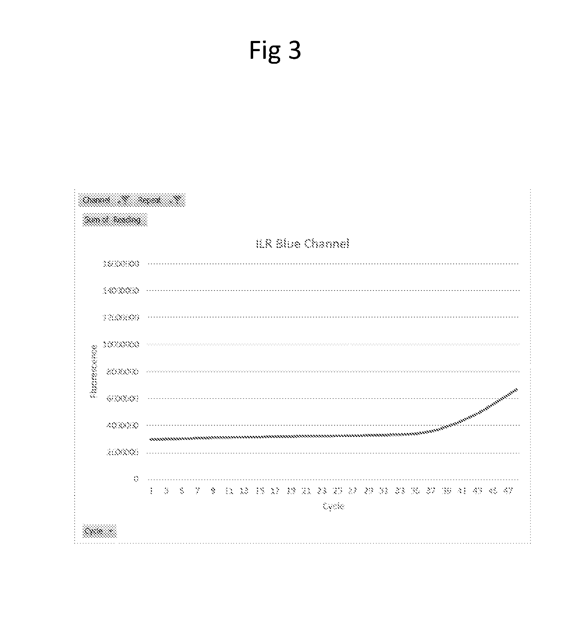

PCR was then performed (details below) with in-line reader (ILR) (also referred to as a fluorometer) measurements of fluorescence, in the FAM channel, of the HBB target taken (see the graph in Figure 3 which is a PCR trace of FAM channel showing commencement of PCR curve).

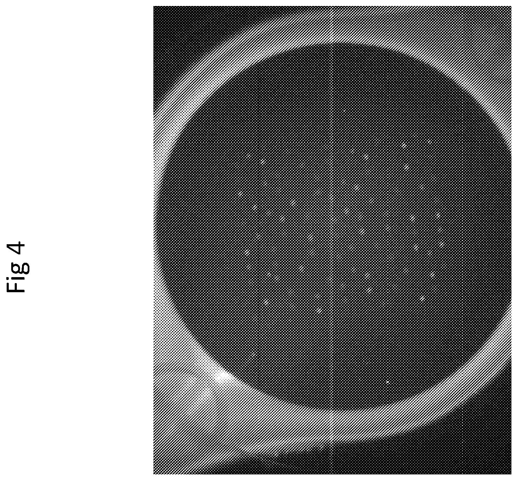

The PCR reaction product was then passed over the microarray, washed in 1x PBS-Tween 20 - 10 mM sodium phosphate, 0.15 M NaCl, 0.05% Tween™ 20 buffer at pH 7.5 and imaged (as shown in Figure 4). Camera exposure: 1000mA LED excitation, CMOS exposure 400ms.

The PCR cycling and array conditions were as follows:

| Step | Time (seconds) | Temperature (°C) | Cycles |
|---|---|---|---|
| Initial Denaturation | 60 | 98 | 1x |
| Cycling Denaturation | 2 | 98 | 48x |
| Anneal | 12 | 60 | 48x |
| Microarray | 600 flow time | 55 | 1x |

It would be understood that the above examples of an STI testing method and panel is just one manner in which the methods and devices of the present invention could be used. There are many other targets that could be looked at such as a coronavirus and flu panel where further particularisation of strains could be provided if a pathogen is present, TB testing panels with further detail of strains and in particular antibiotic resistance information etc.

Additional information

**Primers/probes:** In many embodiments, including the above examples, primary nucleic acid target probes are oligonucleotides that are covalently labelled with a quencher group and fluorophore group. Thereby, while the probe is intact, a fluorophore will not fluoresce when excited, but will rather transfer energy to the quencher group.

During each replication cycle primary nucleic acid target probes are able to anneal to the exposed target sequences (between the primary target primer sites). The probes are cleaved and the fluorophores are de-quenched by the exonuclease action of the polymerase during template extension. The cleaved fluorophore is then able to fluoresce and adds to the total detected signal thereby reporting upon DNA amplification and indirectly the amount of amplicon target present.

The secondary nucleic acid target probes are primer sequences that are covalently labelled with a fluorophore, and do not contain quenchers. The secondary nucleic acid target fluorophores are incorporated directly into the secondary amplicon via the labelled primer during PCR, but the fluorescence signal remains maximally constant throughout PCR. In addition, the unquenched primer-probes concentration is in excess to ensure it is not a rate-limiting factor. The result is that an appreciable absorbance and fluorescence can occur even at wavelengths at the tail ends of the fluorophore excitation and emission spectra, respectively.

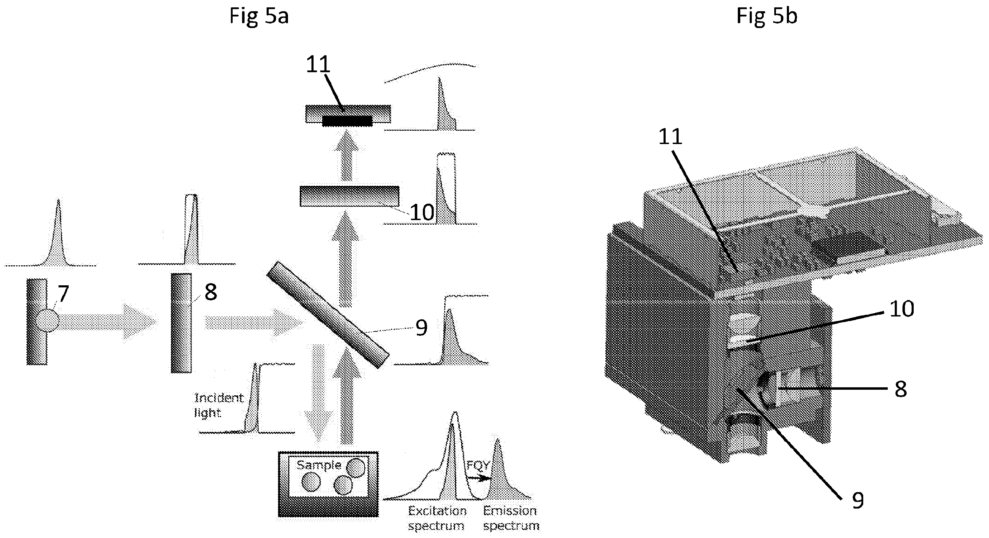

**Fluorometer/qPCR fluorescence reader system (also referred to herein as an in-line reader (ILR)):** An appropriate qPCR fluorescence reader system that can be used in the invention, including in the above examples, comprises of a number, N, of qPCR fluorescence detection channels each consisting of an LED excitation light source **7**, an excitation filter **8**, a dichroic filter **9**, an emission filter **10** and a detector such as a photodiode **11.** The spectral input for an exemplary single excitation/detection channel and fluorophore is shown schematically in Figure 5a with a cross-sectional view showing the physical set up of one light path shown in Figure 5b. The components of each channel are designed to spectrally align with the inherent excitation and emission spectrum of a specific fluorophore to maximize signal the detected fluorescence signal for a given concentration, whilst simultaneously minimising fluorescence crosstalk onto the other N-1 detection channels and minimising leakage of LED light through the system through the spectral separation of the excitation and emission filters and the use of a dichroic mirror.

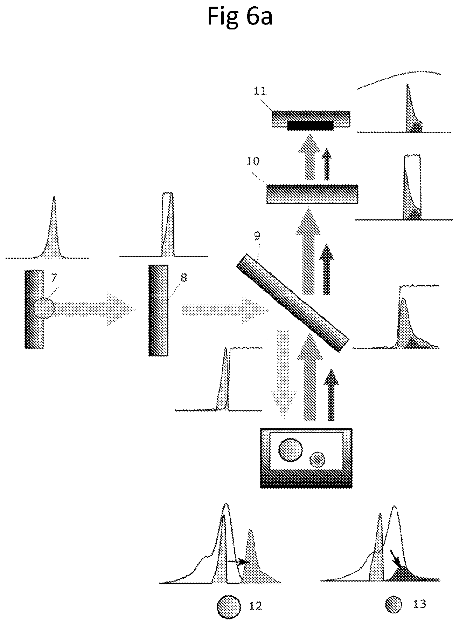

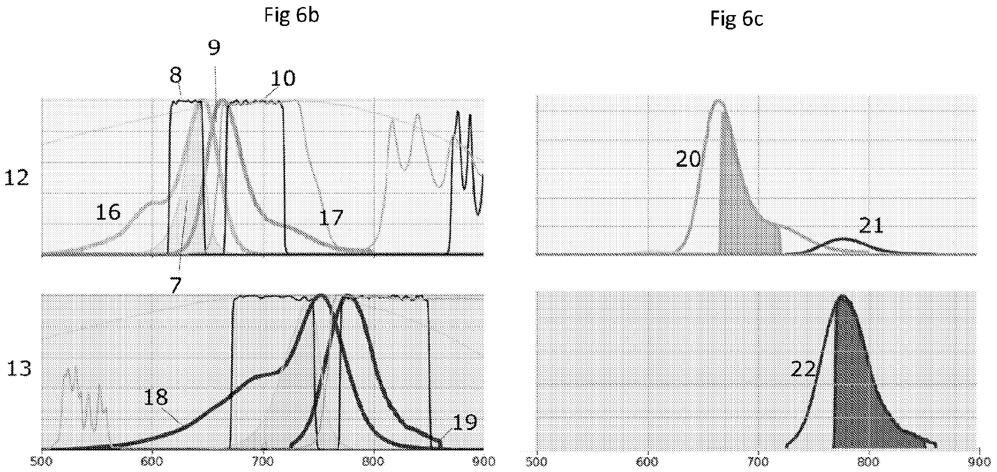

The spectral inputs for an exemplary single excitation/detection channel and two fluorophores are shown schematically in Figure 6a. One fluorophore **12** specific to the channel, contributing the majority of the detectable signal, and a second fluorophore **13**, also present in the mixture and contributing a smaller flux to the overall detectable signal - which we define as the cross-talk component. While there is spectral overlap between the excitation light incident on the sample and the two excitation spectra of both fluorophores, the excitation filter is more aligned with the first fluorophore 12. Likewise while there is spectral overlap between the excitation light incident on the sample and the two emission spectra of both fluorophores, the emission filter is more aligned with first fluorophore 12. The two effects in combination act to minimize crosstalk by limiting the contribution of second fluorophore 13 while ensuring a sufficient signal strength of first fluorophore 12. Figure 6b shows real spectral inputs for two fluorophores (where the first fluorophore 12 is Cy5 and the second fluorophore 13 is AF750) for a potential combination of filters and LEDs. The first fluorophore 12 spectra (fig 6b upper image) are labelled to show the LED spectrum 7, the excitation filter 8, the dichroic mirror 9, and the emission filter 10. The excitation spectra of the two fluorophores are shown as **16** and **18**, and emission spectrum of the two fluorophores are shown as **17** and **19**. Note that while the excitation spectra 16, 18 of both flurophores overlap with the excitation filter 8, the emission filter 10**,** is set specifically to cut-off before emission spectrum of the second fluorophore 19. The result, shown in figure 6c, is that the signal from the on-channel fluorophore dominates due to the greater radiant power absorbed and is better aligned within the emission bandpass **20**, whereas there is negligible crosstalk **21** from the off-channel fluorophore. Thus the combination the two LEDs, filters, fluorophore selection and dichroic mirror can limit crosstalk, while still allowing a strong signal strength on the dedicated channel for second fluorophore 13, as is indicated in the signal within the emission bandpass, **22**. Note that the figure depicts equal concentrations of the unquenched fluorophores in the scaling of the emission spectra. If one fluorophore were in excess of the other the emission spectra in figure 6c would be appropriately scaled.

The micro-array camera can have a similar light path set-up as the in-line reader (also referred to as a fluorometer), but the single photodiode is replaced with a CMOS sensor.

There are three design requirements that must be considered when selecting fluorophores, light sources and filters for a detection channel. These are: firstly, a sufficiently high signal strength, defined as the detected signal due to the fluorophore when excited with its *intended* LED and detecting on its *intended* detection channel; secondly, a sufficient low fluorophore crosstalk, defined as the fluorescence contribution when illuminating the sample with an LED and reading on a detection channel intended for a *different* fluorophore; and thirdly, a sufficient low LED bleed-through, defined as the detection of LED excitation light.

**LED bleed-through:** In the qPCR fluorescence reader system, the dichroic mirror cut-on wavelength is preferably spectrally aligned to fall between the excitation filter cut-off and emission filter cut-on wavelength.

The separation and the reduction of the overlap between these components reduces LED bleed-through when the spectral separation of the excitation filter cut-off and dichroic mirror transmission cut-on wavelengths are sufficiently high and likewise the emission filter cut-on and dichroic mirror transmission cut-on wavelengths are sufficiently high.

For example, bleed-through is reduced when the spectral separation of the excitation filter cut-off and dichroic mirror cut-on wavelengths at the 10% transmittance level is sufficiently high (e.g. above 2nm), and likewise the emission filter cut-on and dichroic mirror cut-on wavelengths at the 90% transmittance level is sufficiently high (e.g. above 2nm).

Further, bleed through can be reduced by further specifying that the out-of-band optical density of an excitation bandpass filter is sufficiently high (e.g. of greater than or equal to 5), particularly within the bandpass of the corresponding emission filter; and likewise that the out-of-band optical density of an emission bandpass filter is sufficiently high (e.g. of greater than or equal to 5), particularly within the bandpass of the corresponding excitation filter. Where in this context the bandpass can be defined as the wavelengths between the 50% cut-on and 50% cut-off edges of the filter, or equivalently the bandwidth centred on the central wavelength (CWL) +/- half of the full width at half maximum (FWHM) of the filter. With this specification in place on the filters and dichroic mirror, LED bleed through the system will be negligible.

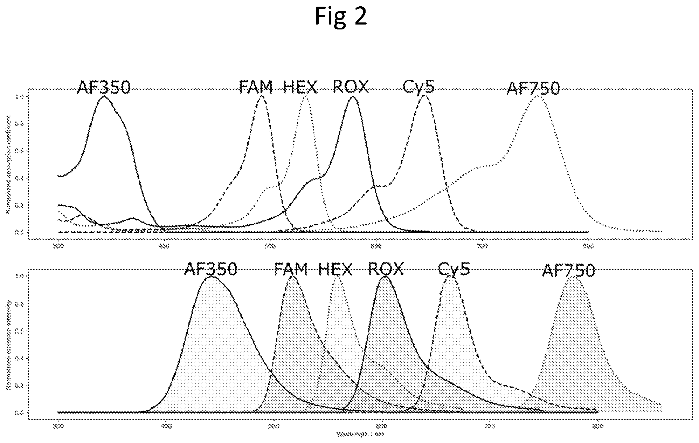

**Crosstalk considerations:** In the present application minimizing crosstalk as far as possible is of particular importance, particularly when larger numbers of primary targets are being assayed. It is possible to reduce crosstalk between fluorophores by filtering LED light to minimal or preferably non-overlap regions of fluorophore excitation spectra and likewise filtering any light emitted by the sample to minimal or preferably non-overlapping regions of fluorophore emission spectra. Narrower bandwidth filters and sharper LED peaks will reduce overlap and hence crosstalk but will also reduce signal strength. In the case of a 6 channel multiplex qPCR experiment some spectral overlap is likely to be inevitable due to the inherent width of fluorophore excitation and emission spectra and the typical wavelength range (UV to infrared) of molecular fluorescence (an indicative spectra for a 6-channel multiplex system is shown in Figure 2).

The fluorophore set will therefore preferably have excitation and emission spectra bandwidths that are spectrally mutually separated (in terms of wavelength) as far as possible.

In the present application, this is specifically important in the case of the fluorophore used to label the plurality of secondary nucleic acid targets and the fluorophores used to probe primary nucleic acid targets.

**Fluorophores:** In many preferred embodiments, including the examples described above, the fluorophore used to label the plurality of secondary nucleic acid targets emits within the longer-wavelength range such as Cy5, AF750 or at a sufficiently short-wavelength in the UV regions, such as AF350.

**Signal strength:** The signal strength is defined as the fluorophore signal detected on its intended channel once passing through the system. In order the fluorophore to fluoresce, light must be transmitted to the sample chamber, therefore the LED emission spectrum and excitation filter bandwidth must spectrally align and overlap to a sufficient extent with the selected fluorophore's excitation spectrum. In order for fluorescence to be detected, light must be transmitted to the detector, therefore the emission filter bandwidth must spectrally align and overlap to a sufficient extent with the selected fluorophore's emission spectrum.

**Crosstalk and signal strength calculation:** It is possible to calculate the expected signal and crosstalk for a system of fluorescence detection channels (incorporating an LED, excitation filter, dichroic mirror, emission filter and sensor) and corresponding fluorophores. Where "on-channel" signal is the contribution of a fluorophore emission to its corresponding detection channel, and the crosstalk is the "off-channel" contribution of a fluorophore to a detection channel other than the one designed for it.

Calculating signal contributions of a fluorophore to a detector reading involves:

- Calculating the expected proportion of light energy that will reach the sample window for each channel (denoted i) for a given LED energy flux. This is determined by the efficiency of the excitation branch of the channel incorporating the LED, excitation filter and dichroic mirror. (equation 1)
- Calculating the proportion of this light which will be absorbed by the specified fluorophore, (denoted j) in the mix. This is determined by the sample concentration and inherent properties of the fluorophore. (equation 2)
- Calculating the proportion of the excited light which be re-emitted by the fluorophore (denoted j). This is determined by the inherent properties of the fluorophore. (equation 3)
- Calculating the proportion of the emission that will be detected by the sensor of the specified channel (denoted i). This is determined by the efficiency of the emission branch of the channel incorporating the dichroic mirror, emission filter and detector sensitivity. (equation 4)
- Finally, the total signal detected on a channel (denoted i) can be calculated by summing all the contributions fluorophores (denoted j) in the sample. (equation 5) This calculation can take the form of a matrix equation (equation 6). Where the matrix elements (equation 7) that are on the diagonals correspond to the on-channel signal contributions (where i = j) and the off diagonal corresponding to the cross-talk contributions (where i ≠ j).

**Equations for crosstalk and signal strength calculation noted above:** The spectral radiant power on the sample is:

$$P_{\lambda\,i}^{\text{incident}} \approx P_i^{\text{led total}} \frac{I_{\lambda\,i}^{\text{led ref}}}{\int I_{\lambda\,i}^{\text{led ref}} d\lambda} \Omega_i^{\text{led}} \xi_{\lambda\,i}^{\text{exf}}(1 - \xi_{\lambda\,i}^{\text{dic}}). \qquad \text{(equation 1)}$$

The integral radiant power absorbed is:

$$P_{i\,j}^{\text{absorbed}} = \int (P_{\lambda\,i}^{\text{incident}}(1 - T_{\lambda\,j})) d\lambda \approx \int (P_{\lambda\,i}^{\text{incident}} c_j\, l\, \ln(10) \varepsilon_{\lambda\,j}) d\lambda. \qquad \text{(equation 2)}$$

The emitted spectral radiant power is:

$$P_{\lambda\,i\,j}^{\text{emission}} = \Upsilon_{\Theta\,j} P_{i\,j}^{\text{absorbed}} \frac{P_{\lambda\,j}^{\text{emission ref}}}{\int P_{\lambda\,j}^{\text{emisson ref}} d\lambda}. \qquad \text{(equation 3)}$$

The total signal intensity contribution detected is:

$$F_{i\,j}^{det} = \int (P_{\lambda\,i\,j}^{\text{emission}} \xi_{\lambda\,i}^{\text{dic}} \xi_{\lambda\,i}^{\text{emf}} \zeta_{\lambda}^{\text{det}}) d\lambda. \qquad \text{(equation 4)}$$

The total signal on the detector can be written as:

$$F_{i}^{\text{det}} = \sum_j F_{ij}^{\text{det}} = P_i^{\text{led total}} \sum_j (A_{ij} c_j) \qquad \text{(equation 5)}$$

$$\boldsymbol{F}^{det} = \text{diag}(\boldsymbol{P}^{\text{led total}})\boldsymbol{Ac} \qquad \text{(equation 6)}$$

Where the on and off-channel signal contributions for unit concentration and LED power are:

$$A_{ij} = \kappa_i \alpha_j \int [\varepsilon_{\lambda\,j}^{\text{ref}} \xi_{\lambda\,i}^{\text{exf}} (1 - \xi_{\lambda\,i}^{\text{dic}}) \frac{I_{\lambda\,i}^{\text{led ref}}}{\int I_{\lambda\,i}^{\text{led ref}} d\lambda}] d\lambda \int [\frac{P_{\lambda\,i\,j}^{\text{emission ref}}}{\int P_{\lambda\,i\,j}^{\text{emission ref}} d\lambda} \xi_{\lambda\,i}^{\text{dic}} \xi_{\lambda\,i}^{\text{emf}} \zeta_{\lambda}^{\text{det}}] d\lambda) \qquad \text{(equation 7)}$$

Where:

- i denotes the excitation and corresponding fluorescence detection channel.

- j denotes the fluorophore.

- $\lambda$ is the wavelength.

- $I_{\lambda\,i}^{\text{led ref}}$ is the reference spectral intensity of the LED (of channel i)

- $\int I_{\lambda\,i}^{\text{led ref}} d\lambda$ is a normalization factor to scale the reference LED spectrum (of channel i).

- $\frac{I_{\lambda\,i}^{\text{led ref}}}{\int I_{\lambda\,i}^{\text{led ref}} d\lambda}$ is the normalized intensity spectrum of the LED (of channel i)

- $\Omega_i^{\text{led}}$ is the solid angle integral of a cone defined by the viewing angle $\theta_i^{\text{led}}$ of the LED (of channel i). Where $\Omega_i^{\text{led}} = 2\pi(1 - \cos(\theta_i^{\text{led}}))$ that assumes $\frac{d^2 I}{d\Omega^2} \approx 0$ within the conic region and that all light within the region reaches the excitation filter. $\Omega_i^{\text{led}}$ is a proportionality constant.

- $\theta_i^{\text{led}}$ is the azimuth viewing angle of half-maximum intensity of the LED (of channel i)

- $P_i^{\text{led total}}$ is the integral radiant power of the LED (of channel i), which is proportional to the current at which it is driven by the device.

- $\xi_{\lambda\,i}^{\text{exf}}$ is the spectral transmittance of the excitation bandpass filter (of channel i)

- $\xi_{\lambda\,i}^{\text{dic}}$ is the spectral transmittance of the dichroic mirror (of channel i)

- $P_{\lambda\,i}^{\text{incident}}$ is the spectral radiant flux incident on the sample (for channel i)

- $l$ is the path length, it is constant for all channels as the depth of the sample chamber

- $c_j$ is amount concentration of the fluorophore (denoted j) in the sample

- $\varepsilon_{\lambda\,j}$ is the spectral molar decadic absorption coefficient of the fluorophore (denoted j), it is calculated from reference fluorophore absorption (excitation) spectra, $\varepsilon_{\lambda\,j}^{\text{ref}}$ by scaling the peak to $\varepsilon_{\max\,j}$ such that $\varepsilon_{\lambda\,j} = \varepsilon_{\max\,j}\varepsilon_{\lambda\,j}^{\text{ref}}$

- $T_{\lambda\,j}$ is the spectral internal hemispherical transmittance of the fluorophore (denoted j) where $T_{\lambda j} = 10^{-c_j l \varepsilon_{\lambda j}}$ but can be approximated for small concentrations by the first two terms a Maclaurin series expansion, $T_{\lambda j} = 1 - \ln(10)\,c_j l \varepsilon_{\lambda j} + \cdots$

- $\Upsilon_{\Theta\,j}$ is the temperature-corrected fluorescent energy yield of the fluorophore (denoted j) it related to the fluorescent quantum yield by $\Upsilon_{\Theta j} = \dfrac{\bar{\lambda}^{excitation}}{\bar{\lambda}^{emission}}\,\Phi_{\Theta j}$ where the flux-weighted wavelengths are defined as $\bar{\lambda}^{excitation} = \dfrac{\int \lambda \varepsilon_\lambda^{ref}\,d\lambda}{\int \varepsilon_\lambda^{ref}\,d\lambda}$ and $\bar{\lambda}^{emission} = \dfrac{\int \lambda P_\lambda^{emission\,ref}\,d\lambda}{\int P_\lambda^{emission\,ref}\,d\lambda}$

- $\Phi_{\Theta j}$ is the temperature-corrected fluorescent quantum yield (FQY) of the fluorophore (denoted j). It can be calculated from literature FQY values, $\Phi_j^{\text{ref}}$, when brightness vs temperature relationships are available using $\Phi_{\Theta j} = \Phi_j^{\text{ref}}k(\Theta)$. Where the correction factor, k($\Theta$), should correspond to the temperature, $\Theta$, at which readings are taken.

- $P_{i\,j}^{\text{absorbed}}$ is the integral radiant power absorbed by the fluorophore (denoted j)

- $P_{\lambda\,j}^{\text{emission ref}}$ is the reference emission spectrum of the fluorophore (denoted j)

- $\int P_{\lambda\,j}^{\text{emisson ref}}d\lambda$ is a normalization factor to scale the reference fluorophore emission spectrum (denoted j).

- $\dfrac{P_{\lambda\,j}^{\text{emission ref}}}{\int P_{\lambda\,j}^{\text{emisson ref}}d\lambda}$ is the normalized emission spectrum of the fluorophore (denoted j)

- $P_{\lambda\,i}^{\text{emission}}$ is spectral radiant power emitted by the fluorophore (denoted j)

- $\xi_{\lambda\,i}^{\text{emf}}$ is the spectral transmittance of the emission bandpass filter (of channel i)

- $\zeta_\lambda^{\text{det}}$ is the wavelength-dependent detector efficiency.

- $F_{ij}^{\text{det}}$ is the contribution of fluorophore (denoted j) to the detected signal (of channel i)

- $F_i^{\text{det}}$ is the reading from the detector (of channel i)

- $\kappa_i = \Omega_i^{\text{led}}l\,ln(10)$ is the proportionality constant of channel i accounting for any scattering effects through the optical system. If all LEDs have similar radiation patterns and scattering effects are channel independent the term can be ignored.

- $\alpha_j = \varepsilon_{maxj}\Upsilon_{\Theta j}$ is the brightness proportionality constant of fluorophore j. This can verified and calibrated empirically for the specific fluorophore set in the system.

- $A_{ij}$ is the concentration and LED power independent signal contribution of fluorophore j to the signal detected on channel i.

- $\boldsymbol{F}^{det}$ is a column vector of readings on all channels with elements $F_i^{\mathrm{det}}$

- $\boldsymbol{c}$ is a column vector of concentrations of all fluorophores with elements $c_j$

- $\boldsymbol{P}^{led\ total}$ is a vector of LED radiant powers used on all channels with elements $P_i^{\mathrm{led\ total}}$

- $\boldsymbol{A}$ is the square matrix of signal strengths and crosstalk contributions with elements $A_{ij}$

The total signal detected on any given channel will be a linear combination of the concentrations of the unquenched primary and/or secondary fluorophore probes present in the sample weighted according to their on-channel and off-channel contributions to the signals (equations 5 and 6). When crosstalk is significant either due to an inherently high crosstalk contribution ($A_{ij}$ where $j \neq i$ in equation 7) or due to a high concentration of an off-channel fluorophore (high $c_j$ where $j \neq i$ in equation 5) this increases the risk of misinterpreting multiplex qPCR data.

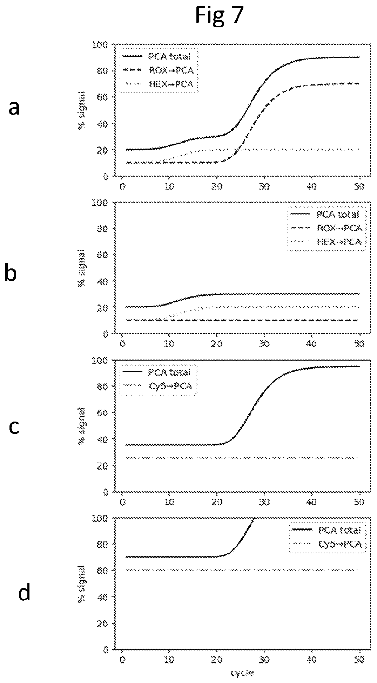

**Crosstalk between primary target probe fluorophores:** It is unlikely in a reasonably well-designed system (where $<< A_{ii}$ when $j \neq i$, defined above in equation 7) that crosstalk between primary probe fluorophores would result in dynamic range issues due to crosstalk from off-channels (j) due to large signal $c_j$. This is because even if $c_i(t) < c_j(t)$ at a given PCR cycle point t, the effective concentration difference is capped by the total amount of probe present, and this will not overcome the inherent crosstalk ratios designed into the system. Rather, the risks in the case where two sets of primary fluorophore probes are present is that it is possible to misinterpret a partial signal on channel i as shown in figure 7a (solid black line) and 7b (solid black line), which arises due to the off-channel amplification of a primary target reporting on a separate channel for which there is a non-zero cross-talk contribution as shown in graph 7a (dotted grey lines) and 7b (dotted grey lines). In one case where there is a true on-channel signal, 7a (black dashed line) there is no miscall, however when there is only a crosstalk 7b (dashed grey line) contribution this may result in a false positive call for the primary probe target i. To mitigate this risk, criteria can be introduced to account for known system crosstalk contributions ($A_{ij}$, defined above in equation 7) by either increasing the required confidence thresholds with which to call a positive based on off channel readings or correcting signals based on off-channels. Both approaches effectively address the same problem. If concentrations of probes are high on several channels a more rigorous approach is to recover the on-channel signals ($F_{jj}^{\mathrm{det}} \propto c_i$, defined above in equation 4) through inversion of matrix equation 6. However, if channels are saturated or the crosstalk matrix A (defined above) cannot be characterised to sufficient precision this may not be possible.

**Risks associated with crosstalk between fluorophores of primary target probes and labelled secondary target primer-probes:** Where both primary target probes and unquenched secondary target primer-probes are present in the same reaction mix, there is a risk that the dynamic range of the primary fluorophore channels will be reduced. In all cases if a qPCR detection channel exists that matches the secondary target fluorophore, the detection channel will be inevitably and unavoidably saturated. Thus, for a 6-channel system where the fluorophore used to label the secondary probe matches one of the primary probe detection channels, in the best case only a 5-plex qPCR will be possible when used in conjunction with an array assay. However, due to non-zero crosstalk components, saturation and severely reduced dynamic range may also occur on other primary channels. The system can however be designed to limit this crosstalk components, to limit the effect to the minimum number of channels and to ensure compatibility with (1) the concentration levels required for the unquenched secondary target primers and (2) the typical LED powers at which primary target fluorophore channels are driven to achieve appreciable amplification signals.

In systems, such as those of the present invention, which must support both primary target (qPCR) and secondary target (microarray) assays the balance between minimizing crosstalk and maximizing expected signal is shifted. When a system only supports primary target probes the weighting of these two criteria may be in favour of maximizing signal as long as some known crosstalk limit is not exceeded on any channel (e.g., $F^{det}_{ii}/F_{jj}^{det} = 0.01$ crosstalk when $c_i = c_j$, or equivalently $F^{det}_{ii}/F_{jj}^{det} = 0.1$ when $c_i = 10c_j$, see equation 4 above). However, for a system where there are large differences between concentrations such as in the present application it is preferable that a greater constraint be put on the acceptable crosstalk component ($A_{ij}$ defined above in equation 7) in as many channels as possible. This is because $c_i >> c_j$ by several orders of magnitude, necessitating reduced crosstalk components at the expense of signal on some channels (e.g. $F_{ii}/F_{ij} < 0.001$ crosstalk when $c_i = c_j$, or equivalently $F_{ii}/F_{ij} < 10\%$ when $c_i = 10^4 c_j$, see equation 4 above) to avoid saturation and retain sufficient dynamic scope to allow the primary probes to report on amplification as shown in figure 7c. However, if either the crosstalk component or required secondary target primer-probe concentration is higher, then saturation will occur, as shown in figure 7d, which may prevent detection of the primary target sequence. The specific constraints that should be put on the maximum crosstalk between channels will depend on the chemistry of the diagnostic tests, the required multiplexing capabilities when simultaneously testing for secondary and primary targets, and the required analytical sensitivity on individual channels (which demands higher $A_{ii}$ components), and optimising the constraints based on the above could be carried out by one skilled in the art without requiring undue experimental burden.

Since the signal contribution on all channels due to the labelled secondary target primers remains constant throughout the qPCR reaction (dashed grey lines in figs 7c and 7d) there is no associated risk of calling a false positive result due to apparent amplification curves mirroring the amplification on the secondary target amplicon.

**Expanding on desired crosstalk ratios:** Advantageously, filter sets comprising emission filters and excitation filters can be used to ensure the emission cross talk is less than 10% of the total signal, preferably less than 5% of the total signal, preferably less than 1% of the total signal, preferably less than 0.1% of the total signal.

Above the $\frac{F_{ij}^{det}}{F_{ii}^{det}} = 10\%$ crosstalk level at any concentration ratio of analytes the risk of miscalling false positives can become unacceptable when considering two multiplexed primary target probes that are cross-talking. Systems with crosstalk at this level can correct the detected signal on any channel with such crosstalk contributions from fluorophores not specific to the channel. The correction can either be applied as a linear combination of the cross-talking signals or by implementing more stringent classification criteria in response to high fluorophore signal that is known to cause crosstalk. This is necessary because the apparent curves due to amplification may have a similar profile to true positive results at low LoD levels.

At the 1% level crosstalk can be discarded when considering only multiplex primary target probes, but if a 1% crosstalk which occurs at equal concentrations is extrapolated to the concentration differences present when secondary and primary target probes are used in combination this can in some cases cause a significant drop in the dynamic range and possibly saturation on the primary target probe channel. It is therefore generally preferred that the emission cross talk is less than 1% of the total signal, more preferably less than 0.1% of the total signal, most preferably less than 0.01% of the total signal.

In order to confidently not knock out channels when concentrations of the secondary target primer are elevated to several fold the crosstalk contributions should be correspondingly low e.g. below a crosstalk contribution of $A_{ij}/A_{ii} = 0.01\%$ level when concentrations are equal, which becomes the dominant contributing fluorophore signal at when the concentration ratio between the secondary and unquenched primary fluorophores exceeds $10^4$. Where $A_{ij}$ is defined in equation 7.

**Ranking strategy to optimize filter and fluorophore selection:** There are three design requirements that can be considered when selecting fluorophores, light sources and filters for a detection channel. These are: firstly, a sufficiently high signal strength, secondly, a sufficient low fluorophore crosstalk, and thirdly, a sufficient low LED bleed-through.

If a set of candidate superset of fluorophores have been selected and characterized such that relative brightness information is available the model described in equation 7 can be used to carry out an *in silico* evaluation of potential optical system combinations.

LED bleed through can be managed by applying simple criteria, for example on the out-of-band OD of the bandpass filters and by enforcing adequate spectral separation between the filters and the dichroic mirror cut-on point.

Following pre-selection of sensible combinations of filters and LED the model can calculate:

- Crosstalk as given by equation 7, where $A_{ij}$ are off-diagonal element, so $i \neq j$
- Take the maximum crosstalk component that affects each channel
- Signal strength given by equation 7, where $A_{ii}$ are diagonal elements, so $i = j$
- In the context of the present application, determines the number of channels that would be made unusable at a specified concentration (e.g. $10^5$) if a specified fluorophore were chosen to label the secondary nucleic acid targets (e.g. Cy5) (e.g. $c_i/c_j = 10^5$ where $c_i$ corresponds to the secondary target primer concentration at the end point).
- Ensures that a minimum level of performance is achieved across all channels by comparing to the maximally performant example in each aspect (e.g. with crosstalk characteristics below 200% of the reference and signal strength above 80%) or based on hard threshold (e.g. a maximum $A_{ij}/A_{ii}$ crosstalk of 1%, more preferably a maximum $A_{ij}/A_{ii}$ crosstalk of 0.1% from any fluorophore onto a channel). Where $A_{ij}$ is defined in equation 7.
- The channels for the remaining candidate systems are binned by crosstalk and signal strength as well as the concentration at which the channels remain unsaturated when secondary probes are present.

## Claims

1. A method for analysing a plurality of nucleic acid targets in a single sample volume by:

   amplifying at least one primary nucleic acid target in a reaction volume using real-time polymerase chain reaction (qPCR) and monitoring the resulting primary amplicon generation in real-time;

and
amplifying a plurality of different secondary nucleic acid targets in the same reaction volume using an asymmetric polymerase chain reaction and analysing the resulting secondary amplicons on a microarray, wherein the amplification of the at least one primary nucleic acid target and the amplification of the plurality of secondary nucleic acid targets occurs substantially simultaneously in the same reaction volume.

2. A method for analysing a plurality of nucleic acid targets as in Claim 1 wherein, where the target is an ribonucleic acid (RNA) target, the method includes the step of reverse transcription of RNA to DNA prior to amplification.

3. A method for analysing a plurality of nucleic acid targets as in Claims 1 or 2 wherein a plurality of different primary nucleic acid targets are amplified in a reaction volume to give a plurality of groups of different primary amplicons.

4. A method for analysing a plurality of nucleic acid targets as in any of the previous claims wherein, in the step of amplifying a plurality of primary nucleic acid targets each resulting group of primary amplicons is labelled with a different fluorophore or is labelled using fluorescently labelled oligonucleotide probes complementary to said resulting group of primary amplicons.

5. A method for analysing a plurality of nucleic acid targets as in claim 4 wherein the method includes the step of detecting fluorescence emission of each of the fluorophores upon excitation.

6. A method for analysing a plurality of nucleic acid targets as in any of claims 3-5 wherein each primary amplicon group is detectable by a different probe, said probe, when intact, having a complementary sequence portion which is complementary to part of the amplicon, and being covalently attached to both a fluorophore and a quencher.

7. A method for analysing a plurality of nucleic acid targets as in any of the previous claims wherein, when monitoring the resulting primary amplicon generation in real-time, the detection of primary amplicons is carried out at least once every thermocycle.

8. A method for analysing a plurality of nucleic acid targets as in any of the previous claims wherein the amplicon groups expanded from the plurality of secondary nucleic acid targets are all labelled with the same fluorophore and the fluorophore used to label the plurality of secondary nucleic acid target amplicons is different to the one or more fluorophores used to label the one or more primary nucleic acid targets.

9. A method for analysing a plurality of nucleic acid targets as in any of the previous claims wherein at least one of each primer pair used to amplify the plurality of secondary nucleic acid targets are labelled with the same fluorophore and the primer that is labelled with a fluorophore is in excess to the other primer in a pair.

10. A method for analysing a plurality of nucleic acid targets as in any of claims 4-9 wherein the fluorophore used to label the plurality of secondary nucleic acid targets is Cy5 or UV fluorophore AF350 and when the fluorophore used to label the plurality of secondary nucleic acid targets is Cy5, fluorophores used to label primary nucleic acid targets are one or more selected from AF-350, FAM, HEX , ROX and AF-750.

11. A method for analysing a plurality of nucleic acid targets as in any of claims 8-10 wherein the fluorophore used to label the plurality of secondary nucleic acid targets is selected to have an excitation spectrum that has limited or no overlap with the excitation spectra of the fluorophores used to label primary nucleic acid targets within the region of the excitation filter bandwidth specific to the fluorophores used to label the primary nucleic acid target probes and/or the fluorophore used to label the plurality of secondary nucleic acid targets is selected to have an emission spectrum that has limited or no overlap with the emission spectra of the fluorophores used to label primary nucleic acid targets within the region of the emission filter bandwidth specific to the fluorophores used to label the primary nucleic acid target probes.

12. A method for analysing a plurality of nucleic acid targets as in any of claims 8-11 wherein the maximum absorption and emission wavelength of the fluorophore used to label the plurality of secondary nucleic acid targets is spectrally (in terms of difference in wavelength) separated from the other wavelengths of the fluorophores used to label primary nucleic acid targets.

13. A method for analysing a plurality of nucleic acid targets as in any of claims 8-12 wherein the fluorophore used to label the plurality of secondary nucleic acid targets is selected to have a high extinction coefficient and fluorescent quantum yield and/or is selected to have an emission spectrum higher or significantly lower than all of the emission spectrums of the fluorophores used to label primary nucleic acid targets and/or is selected to be excitable at a wavelength that has limited or no overlap with the excitation wavelength spectrum of the fluorophores used to label primary nucleic acid targets.

14. A method for analysing a plurality of nucleic acid targets as in any of claims 8-13 wherein the fluorophores used to label primary nucleic acid targets are selected to be excitable at wavelengths that have limited or no overlap with the excitation wavelength spectrum of the fluorophore used to label the plurality of secondary nucleic acid targets such that two or less, preferably one or less, most preferably none, of the primary target fluorophore channels will experience crosstalk from the secondary target fluorophore.

15. A microfluidic device and qPCR and asymmetric PCR reagents for carrying out the method of any of claims 1-14 comprising;

    a body section with at least one microchannel;
    means for moving a liquid sample through the microchannel;
    the microchannel having an amplification area in which PCR amplification is performed, said area comprising heating portion and a cooling portion through which a sample can cycle, and a first detection window through which fluorescence emission from the sample can be detected during every amplification cycle;
    the microchannel having an array area, downstream of the amplification area, said array area comprising a second detection window through which fluorescence emission can be detected.

## Patentansprüche

1. Verfahren zum Analysieren einer Vielzahl von Nukleinsäurezielen in einem einzigen Probenvolumen durch:

    Amplifizieren mindestens eines primären Nukleinsäureziels in einem Reaktionsvolumen unter Verwendung der Echtzeitpolymerasekettenreaktion (qPCR) und Überwachen der resultierenden primären Amplikonbildung in Echtzeit;
    und
    Amplifizieren einer Vielzahl von verschiedenen sekundären Nukleinsäurezielen im gleichen Reaktionsvolumen unter Verwendung einer asymmetrischen Polymerasekettenreaktion und Analysieren der resultierenden sekundären Amplikons auf einem Microarray,
    wobei die Amplifikation des mindestens einen primären Nukleinsäureziels und die Amplifikation der mehreren sekundären Nukleinsäureziele im Wesentlichen gleichzeitig in demselben Reaktionsvolumen stattfindet.

2. Verfahren zum Analysieren einer Vielzahl von Nukleinsäurezielen nach Anspruch 1, wobei, wenn das Ziel ein Ribonukleinsäure (RNA)-Ziel ist, das Verfahren den Schritt der reversen Transkription von RNA zu DNA vor der Amplifikation umfasst.

3. Verfahren zum Analysieren einer Vielzahl von Nukleinsäurezielen nach Anspruch 1 oder 2, wobei eine Vielzahl von verschiedenen primären Nukleinsäurezielen in einem Reaktionsvolumen amplifiziert wird, um eine Vielzahl von Gruppen von verschiedenen primären Amplikons zu ergeben.

4. Verfahren zum Analysieren einer Vielzahl von Nukleinsäurezielen nach einem der vorstehenden Ansprüche, wobei im Schritt des Amplifizierens einer Vielzahl von primären Nukleinsäurezielen jede resultierende Gruppe von primären Amplicons mit einem anderen Fluorophor markiert wird oder unter Verwendung von fluoreszenzmarkierten Oligonukleotidsonden markiert wird, die zu der resultierenden Gruppe von primären Amplicons komplementär sind.

5. Verfahren zum Analysieren einer Vielzahl von Nukleinsäurezielen nach Anspruch 4, wobei das Verfahren den Schritt des Erfassens der Fluoreszenzemission jedes der Fluorophore bei Anregung umfasst.

6. Verfahren zum Analysieren einer Vielzahl von Nukleinsäurezielen nach einem der Ansprüche 3 bis 5, wobei jede primäre Amplikongruppe durch eine andere Sonde erfasst werden kann, wobei die Sonde, wenn sie intakt ist, einen komplementären Sequenzabschnitt aufweist, der komplementär zu einem Teil des Amplikons ist, und kovalent sowohl an einen Fluorophor als auch an einen Quencher gebunden ist.

7. Verfahren zum Analysieren einer Vielzahl von Nukleinsäurezielen nach einem der vorstehenden Ansprüche, wobei beim Überwachen der resultierenden primären Amplikonbildung in Echtzeit der Nachweis von primären Amplikonen mindestens einmal pro Thermocyclus durchgeführt wird.

8. Verfahren zum Analysieren einer Vielzahl von Nukleinsäurezielen nach einem der vorstehenden Ansprüche, wobei die aus der Vielzahl von sekundären Nukleinsäurezielen expandierten Amplikongruppen alle mit demselben Fluorophor markiert sind und der zur Markierung der Vielzahl von sekundären Nukleinsäurezielamplikonen verwendete Fluorophor sich von dem einen oder den mehreren Fluorophoren unterscheidet, die zur Markierung des einen oder der mehreren primären Nukleinsäureziele verwendet werden.

9. Verfahren zum Analysieren einer Vielzahl von Nukleinsäurezielen nach einem der vorstehenden Ansprüche, wobei mindestens einer von jedem Primerpaar, das zum Amplifizieren der Vielzahl von sekundären Nukleinsäurezielen verwendet wird, mit demselben Fluorophor markiert ist und der Primer, der mit einem Fluorophor markiert ist, im Überschuss zu dem anderen Primer in einem Paar vorliegt.

10. Verfahren zum Analysieren einer Vielzahl von Nukleinsäurezielen nach einem der Ansprüche 4 bis 9, wobei der Fluorophor, der zum Markieren der Vielzahl von sekundären Nukleinsäurezielen verwendet wird, Cy5 oder der UV-Fluorophor AF350 ist, und wenn der Fluorophor, der zum Markieren der Vielzahl von sekundären Nukleinsäurezielen verwendet wird, Cy5 ist, sind die Fluorophore, die zum Markieren von primären Nukleinsäurezielen verwendet werden, einer oder mehrere ausgewählt aus AF-350, FAM, HEX, ROX und AF-750.

11. Verfahren zum Analysieren einer Vielzahl von Nukleinsäurezielen nach einem der Ansprüche 8 bis 10, wobei das Fluorophor, das zur Markierung der Vielzahl von sekundären Nukleinsäurezielen verwendet wird, ausgewählt wird, um ein Anregungsspektrum aufzuweisen, das eine begrenzte oder keine Überlappung mit den Anregungsspektren der Fluorophore aufweist, die zur Markierung der primären Nukleinsäureziele innerhalb des Bereichs der Anregungsfilterbandbreite verwendet werden, die für die Fluorophore spezifisch ist, die zur Markierung der primären Nukleinsäureziele verwendet werden und/oder der Fluorophor, der zum Markieren der Vielzahl von sekundären Nukleinsäurezielen verwendet wird, so ausgewählt ist, dass er ein Emissionsspektrum aufweist, das eine begrenzte oder keine Überlappung mit den Emissionsspektren der Fluorophore aufweist, die zum Markieren der primären Nukleinsäureziele innerhalb des Bereichs der Emissionsfilterbandbreite verwendet werden, die für die Fluorophore spezifisch ist, die zum Markieren der primären Nukleinsäurezielsonden verwendet werden.

12. Verfahren zum Analysieren einer Vielzahl von Nukleinsäurezielen nach einem der Ansprüche 8 bis 11, wobei die maximale Absorptions- und Emissionswellenlänge des Fluorophors, der zum Markieren der Vielzahl von sekundären Nukleinsäurezielen verwendet wird, spektral (in Bezug auf den Unterschied in der Wellenlänge) von den anderen Wellenlängen der Fluorophore getrennt ist, die zum Markieren der primären Nukleinsäureziele verwendet werden.

13. Verfahren zum Analysieren einer Vielzahl von Nukleinsäurezielen nach einem der Ansprüche 8 bis 12, wobei der Fluorophor, der zur Markierung der Vielzahl von sekundären Nukleinsäurezielen verwendet wird, ausgewählt wird, um einen hohen Extinktionskoeffizienten und eine hohe Fluoreszenzquantenausbeute zu haben und/oder ausgewählt wird, um ein Emissionsspektrum zu haben, das höher oder signifikant niedriger ist als alle Emissionsspektren der Fluorophore, die zur Markierung der primären Nukleinsäureziele verwendet werden, und/oder ausgewählt wird, um bei einer Wellenlänge anregbar zu sein, die eine begrenzte oder keine Überlappung mit dem Anregungswellenlängenspektrum der Fluorophore hat, die zur Markierung der primären Nukleinsäureziele verwendet werden.

14. Verfahren zum Analysieren einer Vielzahl von Nukleinsäurezielen nach einem der Ansprüche 8 bis 13, wobei die Fluorophore, die zum Markieren der primären Nukleinsäureziele verwendet werden, ausgewählt werden, um bei Wellenlängen anregbar zu sein, die eine begrenzte oder keine Überlappung mit dem Anregungswellenlängenspektrum des Fluorophors aufweisen, der zum Markieren der Vielzahl von sekundären Nukleinsäurezielen verwendet wird, sodass zwei oder weniger, vorzugsweise einer oder weniger, am meisten bevorzugt keiner der primären Zielfluorophorkanäle ein Übersprechen von dem sekundären Zielfluorophor erfahren werden.

15. Mikrofluidische Vorrichtung und qPCR- und asymmetrische PCR-Reagenzien zur Durchführung des Verfahrens nach einem der Ansprüche 1 bis 14, umfassend:

einen Körperabschnitt mit mindestens einem Mikrokanal;
Mittel zum Bewegen einer Flüssigkeitsprobe durch den Mikrokanal;
wobei der Mikrokanal einen Amplifikationsbereich aufweist, in dem die PCR-Amplifikation durchgeführt wird, wobei der Bereich einen Heizabschnitt und einen Kühlabschnitt umfasst, durch die eine Probe zirkulieren kann, und ein erstes Erfassungsfenster, durch das die Fluoreszenzemission der Probe während jedes Amplifikationszyklus erfasst werden kann;

der Mikrokanal einen Arraybereich stromabwärts des Verstärkungsbereichs aufweist, wobei der Arraybereich ein zweites Erfassungsfenster umfasst, durch das die Fluoreszenzemission erfasst werden kann.

**Revendications**

1. Procédé permettant d'analyser une pluralité de cibles d'acide nucléique dans un seul volume d'échantillon par :

   amplification d'au moins une cible d'acide nucléique primaire dans un volume réactionnel à l'aide d'une réaction en chaîne par polymérase en temps réel (qPCR) et surveillance de la génération d'amplicons primaires résultants en temps réel ;
   et
   amplification d'une pluralité de cibles d'acides nucléiques secondaires différentes dans le même volume réactionnel à l'aide d'une réaction en chaîne par polymérase asymétrique et analyse des amplicons secondaires résultants sur un microréseau,
   dans lequel l'amplification de l'au moins une cible d'acide nucléique primaire et l'amplification de la pluralité de cibles d'acide nucléique secondaires se produisent sensiblement simultanément dans le même volume réactionnel.

2. Procédé permettant d'analyser une pluralité de cibles d'acide nucléique selon la revendication 1 dans lequel, lorsque la cible est une cible d'acide ribonucléique (ARN), le procédé comporte l'étape de transcription inverse de l'ARN en ADN avant l'amplification.

3. Procédé permettant d'analyser une pluralité de cibles d'acide nucléique selon les revendications 1 ou 2 dans lequel une pluralité de cibles d'acide nucléique primaires différentes sont amplifiées dans un volume réactionnel pour donner une pluralité de groupes d'amplicons primaires différents.

4. Procédé permettant d'analyser une pluralité de cibles d'acide nucléique selon l'une quelconque des revendications précédentes dans lequel, dans l'étape consistant à amplifier une pluralité de cibles d'acide nucléique primaires, chaque groupe résultant d'amplicons primaires est marqué avec un fluorophore différent ou est marqué à l'aide de sondes oligonucléotidiques marquées par fluorescence complémentaires dudit groupe résultant d'amplicons primaires.

5. Procédé permettant d'analyser une pluralité de cibles d'acide nucléique selon la revendication 4 dans lequel le procédé comporte l'étape consistant à détecter l'émission de fluorescence de chacun des fluorophores lors de l'excitation.

6. Procédé permettant d'analyser une pluralité de cibles d'acide nucléique selon l'une quelconque des revendications 3 à 5 dans lequel chaque groupe d'amplicons primaires est détectable par une sonde différente, ladite sonde, lorsqu'elle est intacte, ayant une partie de séquence complémentaire qui est complémentaire d'une partie de l'amplicon, et étant liée de manière covalente à la fois à un fluorophore et à un extincteur.

7. Procédé permettant d'analyser une pluralité de cibles d'acide nucléique selon l'une quelconque des revendications précédentes dans lequel, lors de la surveillance de la génération d'amplicons primaires résultants en temps réel, la détection d'amplicons primaires est effectuée au moins une fois par cycle thermique.

8. Procédé permettant d'analyser une pluralité de cibles d'acide nucléique selon l'une quelconque des revendications précédentes dans lequel les groupes d'amplicons développés à partir de la pluralité de cibles d'acide nucléique secondaires sont tous marqués avec le même fluorophore et le fluorophore utilisé pour marquer la pluralité d'amplicons de cibles d'acide nucléique secondaires est différent dudit ou desdits fluorophores utilisés pour marquer l'une ou plusieurs cibles d'acide nucléique primaires.

9. Procédé permettant d'analyser une pluralité de cibles d'acide nucléique selon l'une quelconque des revendications précédentes dans lequel au moins l'une parmi chaque paire d'amorces utilisées pour amplifier la pluralité de cibles d'acide nucléique secondaires est marquée avec le même fluorophore et l'amorce qui est marquée avec un fluorophore est en excès par rapport à l'autre amorce dans une paire.

10. Procédé permettant d'analyser une pluralité de cibles d'acide nucléique selon l'une quelconque des revendications 4 à 9 dans lequel le fluorophore utilisé pour marquer la pluralité de cibles d'acide nucléique secondaires est Cy5 ou le fluorophore UV AF350 et lorsque le fluorophore utilisé pour marquer la pluralité de cibles d'acide nucléique secondaires est Cy5, les fluorophores utilisés pour marquer les cibles d'acide nucléique primaires sont un ou plusieurs fluorophores sélectionnés parmi AF-350, FAM, HEX, ROX et AF-750.

11. Procédé permettant d'analyser une pluralité de cibles d'acide nucléique selon l'une quelconque des revendications 8 à 10 dans lequel le fluorophore utilisé pour marquer la pluralité de cibles d'acide nucléique secondaires est sélectionné pour avoir un spectre d'excitation qui a un chevauchement limité ou nul avec les spectres d'excitation des fluorophores utilisés pour marquer les cibles d'acide nucléique primaires dans la région de la largeur de bande de filtre d'excitation spécifique aux fluorophores utilisés pour marquer les sondes de cibles d'acide nucléique primaires et/ou le fluorophore utilisé pour marquer la pluralité de cibles d'acide nucléique secondaires est sélectionné pour avoir un spectre d'émission qui a un chevauchement limité ou nul avec les spectres d'émission des fluorophores utilisés pour marquer les cibles d'acide nucléique primaires dans la région de la largeur de bande de filtre d'émission spécifique aux fluorophores utilisés pour marquer les sondes de cibles d'acide nucléique primaires.

12. Procédé permettant d'analyser une pluralité de cibles d'acide nucléique selon l'une quelconque des revendications 8 à 11 dans lequel la longueur d'onde d'absorption et d'émission maximale du fluorophore utilisé pour marquer la pluralité de cibles d'acide nucléique secondaires est séparée spectralement (en termes de différence de longueur d'onde) des autres longueurs d'onde des fluorophores utilisés pour marquer les cibles d'acide nucléique primaires.

13. Procédé permettant d'analyser une pluralité de cibles d'acide nucléique selon l'une quelconque des revendications 8 à 12 dans lequel le fluorophore utilisé pour marquer la pluralité de cibles d'acide nucléique secondaires est sélectionné pour avoir un coefficient d'extinction et un rendement quantique fluorescent élevés et/ou est sélectionné pour avoir un spectre d'émission supérieur ou significativement inférieur à tous les spectres d'émission des fluorophores utilisés pour marquer les cibles d'acide nucléique primaires et/ou est sélectionné pour être excitable à une longueur d'onde qui a un chevauchement limité ou nul avec le spectre de longueur d'onde d'excitation des fluorophores utilisés pour marquer les cibles d'acide nucléique primaires.

14. Procédé permettant d'analyser une pluralité de cibles d'acide nucléique selon l'une quelconque des revendications 8 à 13 dans lequel les fluorophores utilisés pour marquer les cibles d'acide nucléique primaires sont sélectionnés pour être excitables à des longueurs d'onde qui ont un chevauchement limité ou nul avec le spectre de longueur d'onde d'excitation du fluorophore utilisé pour marquer la pluralité de cibles d'acide nucléique secondaires de telle sorte que deux ou moins, de préférence une ou moins, et le plus préférablement aucun, des canaux de fluorophore de cible primaire ne subira de diaphonie provenant du fluorophore de cible secondaire.

15. Dispositif microfluidique et réactifs qPCR et PCR asymétrique permettant de mettre en œuvre le procédé selon l'une quelconque des revendications 1 à 14, comprenant :

une section de corps avec au moins un microcanal ;
des moyens permettant de déplacer un échantillon liquide à travers le microcanal ;
le microcanal ayant une zone d'amplification dans laquelle l'amplification PCR est effectuée, ladite zone comprenant une partie chauffante et une partie refroidissante à travers lesquelles un échantillon peut circuler, et une première fenêtre de détection à travers laquelle l'émission de fluorescence provenant de l'échantillon peut être détectée pendant chaque cycle d'amplification ;
le microcanal ayant une zone de réseau, en aval de la zone d'amplification, ladite zone de réseau comprenant une seconde fenêtre de détection à travers laquelle l'émission de fluorescence peut être détectée.

Fig 1

Fig 2

# Fig 3

EP 4 479 558 B1

Fig 4

Fig 5a

Fig 5b

Fig 6a

Fig 6b

Fig 6c

Fig 7

EP 4 479 558 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20190262832 A